# FASCICULE DE BREVET EUROPEEN

(11) **EP 0 842 279 B1**
(45) Date de publication et mention de la délivrance du brevet: **31.10.2001**
(21) Numéro de dépôt: 96927107.1
(22) Date de dépôt: 30.07.1996
(51) Int. Cl.: C12N 15/56, C12N 7/01, C12N 5/10, A61K 48/00

(54) **VIRUS AUXILIAIRES POUR LA PREPARATION DE VECTEURS VIRAUX RECOMBINANTS**
HILFSVIRUS FÜR DIE HERSTELLUNG VON REKOMBINANTEN VIRUSVEKTOREN
HELPER VIRUSES FOR PREPARING RECOMBINANT VIRAL VECTORS

(30) Priorité: 31.07.1995 FR 9509289
(43) Date de publication de la demande: 20.05.1998
(60) Demande divisionnaire: 01108475.3
(73) Titulaire: TRANSGENE S.A., 67000 Strasbourg (FR)
(72) Inventeur: LUSKY, Monika, 07910 Freiburg (DE); MEHTALI, Majid, 67400 Illkirch Graffenstaden (FR)
(74) Mandataire: Warcoin, Jacques
(86) Numéro de dépôt international: FR9601200
(87) Numéro de publication internationale: WO9705255

(56) Documents cités:
- EP-A- 0 300 422
- EP-A- 0 488 528
- FR-A- 2 707 664
- J. MOL. BIOL. (1993), 230(1), 174-85 CODEN: JMOBAK;ISSN: 0022-2836, XP000568383 AYRES, ELAINE K. ET AL: "Precise deletions in large bacterial genomes by vector-mediated excision (VEX). The trfA gene of promiscuous plasmid RK2 is essential for replication in several Gram-negative hosts"
- PROC. NATL. ACAD. SCI. U. S. A. (1987), 84(24), 9108-12 CODEN: PNASA6;ISSN: 0027-8424, XP002002088 SAUER, BRIAN ET AL: "Site-specific insertion of DNA into a pseudorabies virus vector"
- NUCLEIC ACIDS RES. (1989), 17(1), 147-61 CODEN: NARHAD;ISSN: 0305-1048, XP002002089 SAUER, BRIAN ET AL: "Cre -stimulated recombination at loxP -containing DNA sequences placed into the mammalian genome"

## Description

La présente invention concerne de nouveaux vecteurs auxiliaires (helper en anglais) permettant de complémenter des vecteurs viraux recombinants défectifs, qui présentent la caractéristique d'être pourvus de séquences de recombinaison reconnues par une recombinase. Elle a également pour objet une cellule de complémentation exprimant la recombinase ainsi qu'une méthode de préparation des vecteurs viraux recombinants sous forme de particules virales infectieuses permettant le transfert et l'expression de gènes d'intérêt dans une cellule ou un organisme hôte. L'invention présente un intérêt tout particulier pour des perspectives de thérapie génique, notamment chez l'homme.

La possibilité de traiter les maladies humaines par thérapie génique est passée en quelques années du stade des considérations théoriques à celui des applications cliniques. Le premier protocole appliqué à l'homme a été initié aux Etats Unis en septembre 1990 sur un patient génétiquement immunodéficient en raison d'une mutation affectant le gène codant pour l'Adénine Désaminase (ADA). Le succès relatif de cette première expérimentation a encouragé le développement de nouveaux protocoles de thérapie génique pour diverses maladies génétiques ou acquises (maladies infectieuses et notamment virales comme le SIDA ou cancers). La grande majorité des protocoles décrits jusqu'à présent met en oeuvre des vecteurs viraux pour transférer et exprimer le gène thérapeutique dans les cellules à traiter.

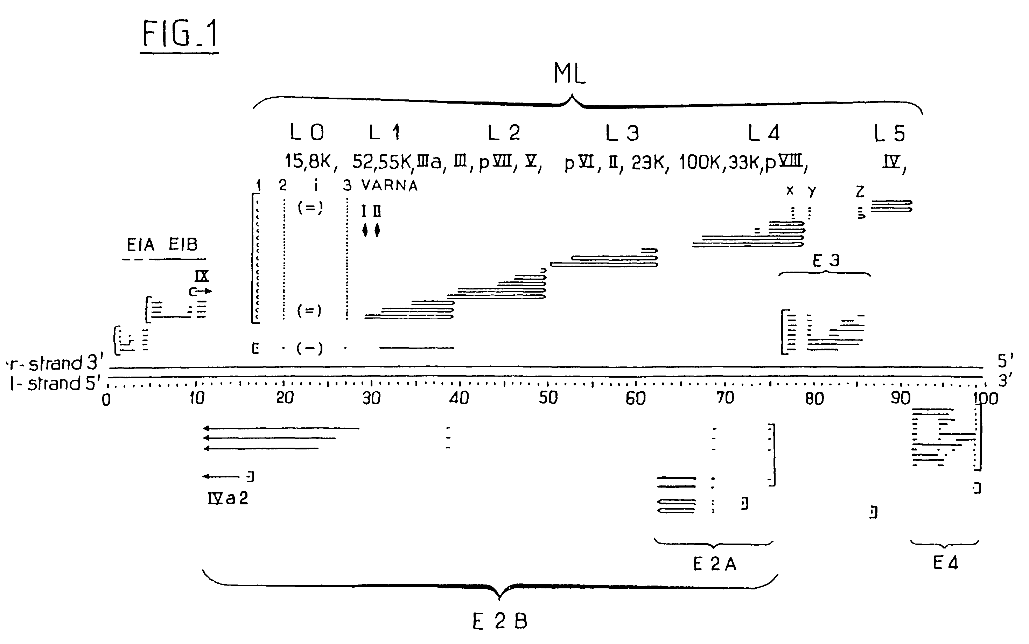

L'intérêt des adénovirus à titre de vecteurs de thérapie génique a déjà été évoqué dans de nombreux documents de l'art antérieur. En effet, les adénovirus ont un large spectre d'hôtes, sont peu pathogènes et ne présentent pas les inconvénients liés aux rétrovirus puisqu'ils sont non-intégratifs et se repliquent également dans les cellules quiescentes. A titre indicatif, leur génome est constitué d'une molécule d'ADN linéaire et bicaténaire d'environ 36 kb portant des régions agissant *en cis* (ITR 5' et région d'encapsidation en 5' du génome viral et ITR 3') et de plus d'une trentaine de gènes, à la fois des gènes précoces nécessaires à la replication virale et des gènes tardifs de structure (voir Figure 1).

Les gènes précoces sont répartis en 4 régions dispersées dans le génome adénoviral (E1 à E4 ; E pour early signifiant précoce en anglais). Elles comportent 6 unités transcriptionnelles qui possèdent leurs propres promoteurs. Les gènes tardifs (L1 à L5 ; L pour late signifiant tardif en anglais) recouvrent en partie les unités de transcription précoces et sont, pour la plupart, transcrits à partir du promoteur majeur tardif MLP (pour Major Late Promoter en anglais).

A l'heure actuelle, tous les vecteurs adénoviraux utilisés dans les protocoles de thérapie génique sont dépourvus de la majeure partie de la région E essentielle à la replication, afin d'éviter leur dissémination dans l'environnement et l'organisme hôte (vecteurs de première génération). Cette délétion rend le génome viral déficient pour la replication. Cependant, les virus E1⁻ peuvent être propagés dans une lignée cellulaire qui complémente la fonction E1 pour générer une particule virale infectieuse. On utilise couramment la lignée 293, établie à partir de cellules de rein embryonnaire humain, dans le génome de laquelle est intégrée l'extrémité 5' gauche de l'adénovirus de type 5 (Graham et al., 1977, J. Gen. Virol. *36*, 59-72).

La plupart des vecteurs adénoviraux de l'art antérieur comportent des délétions supplémentaires. Certaines ont été introduites dans la région E3 dans le but d'accroître les capacités de clonage mais ne nécessitent pas d'être complémentées dans la mesure où la région E3 est non essentielle à la replication. Plus récemment, des vecteurs de seconde génération ont été proposés dans la littérature. Ils conservent les régions *en cis* (ITRs et séquences d'encapsidation) et comportent des délétions internes importantes visant à supprimer l'essentiel des gènes viraux dont l'expression peut être responsable de réponses inflammatoires chez l'hôte. A cet égard, un vecteur minimal déficient pour la totalité des régions virales codantes représente une alternative de choix.

Les techniques de préparation des vecteurs adénoviraux sont largement décrites dans la littérature. Dans un premier temps, le génome complet est constitué par recombinaison homologue dans la lignée 293 (voir notamment Graham et Prevect, 1991, Methods in Molecular Biology, Vol 7, Gene Transfer and Expression Protocols ; Ed E. J. Murray, The Human Press Inc, Clinton, NJ) ou dans *Escherichia coli* (technique décrite dans la demande française No 94 14470).

Il est ensuite nécessaire de propager le vecteur afin de constituer un stock de particules virales le contenant. Cette étape de production est critique et doit permettre d'atteindre des titres élevés en particules infectieuses pour pouvoir envisager un développement à grande échelle en vue de la préparation de lots cliniques. Si les vecteurs adénoviraux de première génération peuvent être propagés relativement facilement dans la lignée cellulaire 293, seule lignée de complémentation décrite à ce jour et capable d'exprimer efficacement E1, tel n'est pas le cas des vecteurs de seconde génération. En effet, selon le même principe de base, un tel vecteur doit être complémenté pour les fonctions essentielles qu'il ne peut exprimer.

La complémentation peut être fournie "*en trans*" par la lignée cellulaire employée (désignée lignée cellulaire de complémentation). Il est alors nécessaire de disposer de nouvelles lignées complémentant plusieurs fonctions virales essentielles (E1 et E2, E1 et E4 ou E1, E2 et E4). Cependant, les diverses tentatives réalisées jusqu'à présent laissent penser que la co-expression de plusieurs régions adénovirales est potentiellement toxique, de sorte que la lignée risque de ne pas être optimale en terme de capacité de croissance et rendement en particules virales, ces deux critères étant indispensables à une exploitation industrielle.

Une autre alternative repose sur l'emploi d'un élément viral supplémentaire, appelé "virus auxiliaire" introduit dans la lignée en même temps que le vecteur adénoviral (système à deux composantes). A l'heure actuelle, on utilise couramment un adénovirus délété de la région E1 capable de synthétiser les produits d'expression des autres régions adénovirales. La co-transfection d'un tel virus auxiliaire et d'un vecteur adénoviral dans la lignée 293 permet la formation de particules virales.

Cependant, un inconvénient majeur de cette méthode est que les cellules produisent une population mixte de particules virales, les unes comportant le vecteur recombinant et les autres le virus auxiliaire. En pratique, les préparations contiennent majoritairement des particules virales de virus auxiliaire, la contamination pouvant atteindre et même dépasser 90%. La présence du virus auxiliaire n'est pas souhaitable dans le cadre d'une thérapie appliquée à l'homme et, de ce fait, nécessite la mise en oeuvre de techniques physiques de séparation lourdes et coûteuses, telles que l'ultracentrifugation. De plus, cette technologie est peu adaptée à la production de vecteurs de structure complexe, tels que les vecteurs de seconde génération, dans la mesure où, très souvent, le virus auxiliaire présente un avantage sélectif (replication plus rapide).

Le problème non résolu à ce jour de la production de particules de vecteur adénoviral recombinant à un titre élevé fait obstacle au développement de la thérapie génique.

On a maintenant construit un nouveau virus auxiliaire par insertion de répétitions directes de part et d'autre de la région d'encapsidation. L'action d'une recombinase les reconnaissant entraîne l'excision du matériel génétique situé entre celles-ci. Cette délétion n'a pas de conséquence notable sur l'expression des gènes viraux mais limite l'encapsidation du virus auxiliaire dans une particule virale. Ainsi, la mise en oeuvre du procédé à deux composantes précédemment décrit dans une lignée cellulaire exprimant la recombinase, permettra de produire des préparations enrichies en particules de vecteur adénoviral d'intérêt.

La présente invention découle de la mise au point d'une technique génétique basée sur l'emploi de séquences de recombinaison et d'une recombinase pour produire majoritairement le vecteur viral recombinant et limiter la contamination par le virus auxiliaire. Le but de la présente invention est de mettre à la disposition du public un nouveau virus auxiliaire capable d'exprimer les gènes qu'il porte (c'est à dire apte à exercer sa fonction de trans-complémentation) mais incapable d'être propagé en présence d'une recombinase. La solution apportée par la présente invention combine sécurité d'emploi (préparation enrichie en vecteur viral recombinant), simplicité (production dans une lignée cellulaire conventionnelle en présence de recombinase) et efficacité ( titre élevé compatible avec les besoins industriels). Elle est tout particulièrement adaptée à la production de vecteurs adénoviraux de seconde génération.

C'est pourquoi la présente invention a pour objet un virus auxiliaire pour la production d'un vecteur viral recombinant défectif pour la replication, caractérisé en ce qu'il comprend une première séquence de recombinaison en 5' et une seconde séquence de recombinaison en 3' d'une région essentielle à la propagation dudit virus auxiliaire ; lesdites séquences de recombinaison étant reconnues par une recombinase.

Le terme "virus auxiliaire" désigne un vecteur capable de trans-complémenter en totalité ou en partie un vecteur viral recombinant défectif pour la replication. Il est donc capable de produire au moins un polypeptide, précoce et/ou tardif, que le vecteur recombinant ne peut lui-même produire et qui est nécessaire à la constitution d'une particule virale. "En totalité" signifie que le virus auxiliaire est apte à complémenter l'ensemble du génome viral essentiel à la replication dont le vecteur viral recombinant est dépourvu et "en partie" signifie que la complémentation est limitée à une partie des fonctions défectives.

Dans le cadre de la présente invention, un virus auxiliaire dérive aussi bien d'un virus naturel tel que trouvé dans la nature que d'un virus dont le génome comporte des modifications par rapport à celui du virus parental dont il est issu. Ces modifications peuvent avoir été introduites *in vitro* par les techniques du génie génétique. Elles peuvent être diverses (délétion, mutation et/ou addition d'un ou plusieurs nucléotides) et localisées dans les régions codantes du génome viral ou en dehors de celles-ci. La modification peut, par exemple, permettre d'inactiver un ou plusieurs gène(s) essentiel(s) à la replication virale dans le but de le rendre également défectif c'est à dire incapable de replication autonome.

Les adénovirus humains de sérotype C et, plus particulièrement, de type 2, 5 ou 7 représentent des virus particulièrement préférés dans le cadre de l'invention. Cependant, on peut également avoir recours à d'autres adénovirus, notamment d'origine animale (canine, bovine, murine, aviaire, ovine, porcine ou simienne). On peut citer plus particulièrement les adénovirus canins CAV-1 ou CAV-2, aviaires DAV ou encore bovins Bad de type 3 (Zakharchuk et al., 1993, Arch. Virol., *128*, 171-176 ; Spibey et Cavanagh, 1989, J. Gen. Virol., *70,* 165-172 ; Jouvenne et al., 1987, Gene, *60*, 21-28 ; Mittal et al., 1995, J. Gen. Virol., *76*, 93-102). Mais, il peut aussi être intéressant de disposer d'un virus auxiliaire dérivant d'un poxvirus (virus de la vaccine, fowlpox, canarypox....), rétrovirus, virus de l'herpès, cytomégalovirus, virus associé à l'adénovirus (AAV) ou encore à d'un virus hybride comportant des fragments de différentes origines.

La caractéristique du virus auxiliaire selon l'invention est qu'il comprend au moins deux séquences de recombinaison insérées en 5' et en 3' d'une séquence essentielle à sa propagation. Une séquence essentielle désigne tout ou partie d'un gène viral, des éléments nécessaires à l'expression comme un promoteur ou, de manière préférée, des élément agissant *en cis* (ITR, LTR, séquence d'encapsidation...). A titre indicatif, les première et/ou seconde séquences de recombinaison peuvent être positionnées à l'intérieur ou immédiatement en 5' et 3' de la région essentielle jusqu'à plusieurs 100aines de pb.

Au sens de la présente invention, une "séquence de recombinaison" est constituée par une séquence d'acide nucléique (ADN ou ARN) reconnue par une recombinase capable d'induire un événement de recombinaison. Habituellement, une séquence de recombinaison a au moins 10 paires de base (pb), avantageusement de 15 à 80 pb, de préférence de 20 à 60 pb et, de manière tout à fait préférée , de 30 à 50 pb. Selon un mode de réalisation avantageux, un virus auxiliaire selon l'invention comprend deux copies d'une séquence de recombinaison identiques ou étroitement apparentées (au moins 70% d'identité de séquence et, de manière préférée au moins 90%). C'est pourquoi on parlera de répétitions de séquences. A cet égard, la répétition peut être inversée (les deux séquence de recombinaison présentes dans le virus auxiliaire ont une orientation réverse l'une par rapport à l'autre, l'une étant dans le sens 5' vers 3' et l'autre 3' vers 5') ou directe (même orientation 5' vers 3' ou 3' vers 5'). On préferera ce second cas de figure.

La recombinaison s'accompagne d'un appariement des séquences de recombinaison, d'un clivage d'une séquence cible à leur niveau et d'une ligation des extrémités clivés L'enzyme capable de promouvoir la recombinaison est désignée "recombinase". La recombinaison entre des répétitions directes conduit à l'excision des séquences comprises entre celles-ci. Par contre, la recombinaison entre des répétitions inversées entraîne l'inversion du matériel génétique localisé entre celles-ci.

D'une manière générale, les séquences de recombinaison et les recombinases sont décrites dans la littérature accessible à l'homme de l'art. Elles peuvent être d'une origine quelconque virale, phagique, procaryote ou eucaryote (levure, champignon ou même eucaryote supérieur). En outre, elles peuvent être obtenues par les techniques conventionnelles de biologie moléculaire (clonage, amplification par réaction de chaîne (PCR pour Polymerase Chain Reaction en anglais) ou par synthèse chimique.

A titre d'exemples préférés, on mentionnera les séquences de recombinaison loxP (décrite dans l'identificateur de séquence SEQ ID NO: 1), FRT (SEQ ID NO: 2) et R (SEQ ID NO: 3) reconnues par les recombinases CRE, FLP et R, respectivement (voir par exemple l'article de revue Kilby et al., 1993, TIG *9*, 413-420).

Un virus auxiliaire particulièrement adapté à la présente invention est dérivé du génome d'un adénovirus et comprend les ITRs 5' et 3', une région d'encapsidation et au moins un gêne viral sélectionné parmi les gènes des régions E1, E2, E4 et L1-L5 et défectif dans le vecteur adénoviral recombinant ainsi qu'une première séquence de recombinaison en 5' et une seconde séquence de recombinaison en 3' de la région d'encapsidation. De préférence, celles-ci,sont constituées par des séquences loxP positionnées dans la même orientation l'une par rapport à l'autre. Selon une première variante intéressante, la région d'encapsidation peut être atténuée (capacité réduite à l'encapsidation) pour favoriser l'encapsidation du vecteur viral recombinant. L'atténuation peut être obtenue par délétion d'une partie de la région d'encapsidation Les moyens d'atténuer une région d'encapsidation sont indiquées dans la demande WO 94/28152

Le virus auxiliaire selon l'invention comprend également une cassette d'expression d'une recombinase, et permettant notamment une expression inductible ou la production d'une recombinase inactive activable en présence d'un ligand hormonal. En effet, dans le cadre de la présente invention la recombinase est une proteine hybride résultant de la fusion d'un polypeptide ayant une activité recombinase et d'un domaine de liaison à un ligand hormonal. L'insertion a lieu dans une région appropriée du virus auxiliaire et, de préférence, en dehors de la région localisée entre les séquences de recombinaison

Selon un mode de réalisation particulier destiné à renforcer l'aspect sécurité, un virus auxiliaire selon l'invention peut comprendre une première séquence de recombinaison et une seconde séquence de recombinaison en 5' et en 3' de plusieurs régions essentielles à sa propagation. Pour des raisons de simplicité de mise en oeuvre, on préférera le cas où les séquences de recombinaison sont identiques ou apparentées de manière à être reconnues par une même recombinase.

La présente invention a également trait à une lignée cellulaire de complémentation pour la production d'un vecteur viral défectif pour la replication comprenant
(i) un premier fragment d'ADN capable de complémenter *en trans* les fonctions défectives d'un tel vecteur, et
(ii) un second fragment d'ADN codant pour une protéine hybride résultant de la fusion d'un polypeptide ayant une activité recombinase et d'un domaine de liaison à un ligand hormonal et capable de reconnaître une séquence de recombinaison.]
Elle peut être générée à partir de diverses lignées cellulaires par introduction de portions appropriées du génome viral et du fragment en question. On préfère tout particulièrement une lignée capable de complémenter la fonction E1 et/ou E4 d'un adénovirus. On mentionnera les lignées 293 (Graham, 1977, *supra*) et 1653 (décrite dans la demande WO 94/28152) modifiées par l'introduction du fragment d'ADN codant pour une recombinase.

Tous les moyens standards pour introduire un acide nucléique dans une cellule peuvent être employés dans le cadre de la présente invention (vecteur synthétique, viral, plasmide , ADN nu...). Bien entendu, ledit fragment d'ADN peut être intégré dans le génome cellulaire ou rester à l'état d'épisome. Aux fins de la présente invention, il peut comprendre les éléments nécessaires à son expression. Il s'agira, de préférence, d'éléments conférant une expression inductible en réponse à un inducteur. De tels éléments sont connus de l'homme du métier On peut citer, à titre indicatif, les promoteurs inductibles par les métaux (promoteur du gène de la métallothionéine), par les hormones (promoteur comprenant des éléments répondant aux glucoconicoïdes GRE, aux progestérones PRE, aux oestrogènes ERE...), par des inducteurs viraux (promoteur comprenant la séquence TAR ou RRE répondant respectivement à la protéine TAT ou REV du virus de l'immunodéficience humaine VIH) ou par divers inducteurs cellulaires (promoteur comprenant la séquence UAS-Gal4 (pour Upstream Activating Sequence Gal4) répondant à Gal4 ou les opérateurs de l'opéron bactérien tétracycline répondant positivement au trans-activateur tétracycline tTA.

Par ailleurs, le second fragment d'ADN en usage dans la présente invention, code pour une recombinase capable de reconnaître les séquences de recombinaison présentes dans un virus auxiliaire selon l'invention. On préfère employer une recombinase sélectionnée parmi le groupe constitué par CRE, FLP et R. Cependant, il est également possible d'avoir recours à un fragment d'ADN codant pour un homologue d'une recombinase dont la séquence est modifiée par rapport à la séquence native mais exerçant une fonction similaire ou améliorée. Ces modifications peuvent résulter de la délétion, addition ou substitution d'un ou plusieurs nucléotide(s). Comme indiqué plus haut, il s'agit, dans le cadre de la présente invention, d'une protéine hybride résultant de la fusion de polypeptides d'origines diverses, notamment d'un polypeptide ayant une activité de recombinase et l'autre d'un domaine de liaison. Une recombinase particulièrement adaptée à la présente invention est constituée par une protéine hybride, désignée CRE-ER, résultant de la fusion de la recombinase CRE et du domaine de liaison au ligand du récepteur oestrogène humain ; (Metzger et al., 1995, Proc. Natl. Acad. Sci. USA *92*). Celle-ci en tant que telle est inactive et son activité biologique est activée en présence d'un ligand hormonal comme l'oestradiol.

L'invention concerne également un procédé pour préparer une particule virale comprenant un vecteur viral recombinant, qui comprend les étapes suivantes :
(a) Préparer un vecteur viral recombinant déficient pour la replication ;
(b) Préparer un virus auxiliaire selon l'invention ;
(c) Introduire le vecteur viral recombinant et le virus auxiliaire dans une lignée cellulaire appropriée ;
(d) Cultiver ladite lignée cellulaire dans des conditions appropriées pour permettre la production de la particule virale en présence d'une recombinase fonctionnelle capable de reconnaître lesdites première et seconde séquences de recombinaison ; et
(e) Récupérer la particule virale dans la culture cellulaire.

Au sens de la présente invention, un vecteur viral recombinant défectif dérive d'un virus dans le génome duquel certaines séquences ont été délétées, rendues non fonctionnelles, mutées ou encore substituées par d'autres séquences et, plus particulièrement, un fragment d'ADN hétérologue (normalement non présent dans le virus parental). L'insertion a lieu dans une région appropriée du génome viral, de manière à permettre son expression dans une cellule hôte. Une cellule hôte est constituée par toute cellule eucaryote infectable par une particule virale contenant ledit vecteur viral recombinant.

Le fragment d'ADN hétérologue en usage dans la présente invention, peut être issu d'un organisme eucaryote, d'un procaryote ou d'un virus autre que celui dans lequel il est inséré. Il peut être isolé par toute technique conventionnelle dans le domaine de l'art, par exemple par clonage, PCR ou synthèse chimique. Il peut s'agir d'un fragment de type génomique (comportant tout ou partie de l'ensemble des introns), de type ADN complémentaire (ADNc, dépourvu d'intron) ou de type mixte (comportant tout ou partie d'au moins un intron). Par ailleurs, il peut coder pour un ARN antisens et/ou un ARN messager (ARNm) qui sera ensuite traduit en polypeptide d'intérêt celui-ci pouvant être (i) intracellulaire, (ii) membranaire présent à la surface de la cellule hôte ou (iii) secrété dans le milieu extérieur. En outre, il peut s'agir d'un polypeptide tel que trouvé dans la nature (natif) ou d'une portion de celui-ci (tronqué) ou également d'un polypeptide chimère provenant de la fusion de séquences d'origines diverses ou encore muté et présentant des propriétés biologiques améliorées ou modifiées.

Dans le cadre de la présente invention, il peut être avantageux d'utiliser un fragment d'ADN codant pour une cytokine (interleukine dont l'IL-2, interféron, facteur stimulateur de colonies...), un récepteur cellulaire ou nucléaire, un ligand, un facteur de coagulation (Facteur VII, Facteur VIII, Facteur IX...), la protéine CFTR (Cystic Fibrosis Transmembrane Conductance Regulator en anglais), l'insuline, la dystrophine, une hormone de croissance, une enzyme (uréase rénine, thrombine...), un inhibiteur d'enzyme (inhibiteur d'une protéase virale, α1-antitrypsine....), un polypeptide à effet antitumoral (produit de gènes supresseurs de tumeurs, polypeptide stimulant le système immunitaire....), un polypeptide capable d'inhiber ou ralentir le développement d'une infection bactérienne, virale ou parasitaire (polypeptide antigénique, variant trans-dominant...), un anticorps, une toxine, une immunotoxine et enfin un marqueur (luciférase, β-galactosidase, produit conférant la résistance à un antibiotique...). Bien entendu, cette liste n'est pas limitative et d'autres gènes peuvent également être employés.

Avantageusement, le fragment d'ADN hétérologue est placé sous le contrôle des éléments nécessaires à son expression dans la cellule hôte. Par "éléments nécessaires", on désigne l'ensemble des éléments permettant la transcription dudit fragment d'ADN en ARN (ARN antisens ou ARNm) et la traduction de l'ARNm en polypeptide. Ces éléments comprennent un promoteur régulable ou constitutif, lequel peut être hétérologue ou au contraire homologue au virus parental. On peut citer, à titre d'exemples, le promoteur du gène PGK (Phospho Glycérate Kinase) humain ou murin, le promoteur précoce du virus SV40 (Simian Virus), le LTR du RSV (Rous Sarcoma Virus), le promoteur TK (Thymidine Kinase) du virus HSV-1 (Herpes Simplex Virus) et les promoteurs adénoviraux E1A et MLP. Les éléments nécessaires peuvent, en outre, inclure des éléments additionnels (séquence intronique, séquence signal de sécrétion, séquence de localisation nucléaire, site d'initiation de la traduction, signal poly A de terminaison de la transcription...). Bien qu'il ne s'agisse pas d'une variante préférée, on indique que le vecteur viral peut également comporter un fragment d'ADN codant pour la recombinase.

Il est à la portée de l'homme du métier de générer un vecteur viral recombinant en usage dans la présente invention. Il saura bien évidemment adapter la technologie en fonction des données spécifiques (type de vecteur, fragment d'ADN hétérologue...). Selon une variante préférée, les vecteurs susceptibles d'être mis en oeuvre dans le cadre de la présente invention, sont des vecteurs adénoviraux recombinants défectifs pour l'ensemble des fonctions virales ou encore toutes les fonctions à l'exception de E4. De tels vecteurs sont décrits dans la demande internationale WO 94/28152.

On obtient un virus auxiliaire selon l'invention par insertion dans un génome viral d'une première et d'une seconde séquence de recombinaison de part et d'autre d'une région essentielle à la replication et, de préférence de la région d'encapsidation celle-ci pouvant être atténuée ou non. L'homme du métier connaît les régions essentielles à la replication d'un virus et est apte à réaliser une telle construction en appliquant les techniques classiques de biologie moléculaire. Selon la variante mentionnée précédemment, il peut également comprendre un fragment d'ADN codant pour une recombinase et, notamment, l'hybride CRE-ER. Selon un mode de mise en oeuvre préférentiel, un virus auxiliaire selon l'invention et le vecteur viral recombinant dont il permet la production, dérivent du même virus parental et, de manière tout à fait préférée, d'un adénovirus.

Après l'étape de construction proprement dite, le virus auxiliaire et le vecteur viral recombinant sont introduits dans une lignée cellulaire appropriée. Tous les moyens standards pour introduire un acide nucléique dans une cellule peuvent être utilisés dans le cadre de la présente invention, par exemple la transfection, l'électroporation, la microinjection, la lipofection, l'adsorption et la fusion de protoplastes. On indique qu'ils peuvent être co-introduits (façon concommittante) ou introduits de manière séparée (le virus auxiliaire selon l'invention préalablement ou postérieurement au vecteur viral recombinant).

Bien que toute lignée cellulaire puisse être employée dans le cadre de la présente invention, on préfère notamment une lignée de complémentation. On aura recours à une lignée de l'art antérieur (293, 1653...) lorsqu'on met en oeuvre un vecteur viral recombinant ou un virus auxiliaire comprenant le fragment d'ADN codant pour la recombinase. En revanche, lorsque ce n'est pas le cas, on utilisera une lignée cellulaire de complémentation selon l'invention.

Après la transfection, la lignée cellulaire est cultivée dans des conditions appropriées pour permettre la production de particules virales. Un procédé selon l'invention peut, en outre, comprendre une étape d'amplification préalablement à l'étape de culture en présence de la recombinase fonctionnelle. Cette étape a pour but d'augmenter les quantités de virus auxiliaire et de vecteur viral recombinant afin d'améliorer les rendements. Elle peut être réalisée par culture dans toute lignée permissive ou dans la lignée appropriée en usage dans la présente invention avant l'ajout, l'expression ou l'activation de la recombinase.

Cette première étape de culture est suivie d'une seconde réalisée en présence d'une recombinase fonctionnelle capable de reconnaître lesdites première et seconde séquences de recombinaison. Dans le cadre du procédé selon l'invention, celle-ci peut être ajoutée à la culture cellulaire, par exemple sous forme substantiellement pure. Toutefois, selon une autre variante tout à fait préférée et déjà évoquée, la recombinase est produite par l'un des constituants du procédé selon l'invention, à savoir le vecteur viral recombinant ou de manière préférée, le virus auxiliaire ou la lignée cellulaire. Une fois produite sous forme fonctionnelle, la recombinase provoquera l'excision de la région essentielle du virus auxiliaire localisée entre les séquences de recombinaison, dans le but d'empêcher ou de réduire sa propagation.

Par ailleurs, lorsqu'on met en oeuvre une recombinase dont l'expression est inductible par un inducteur ou la protéine hybride CRE-ER dont l'activité biologique est dépendante d'un ligand hormonal, l'étape de culture en présence de la recombinase fonctionnelle est réalisée par l'ajout dans le milieu de culture de l'inducteur ou du ligand.

Selon un mode de mise en oeuvre avantageux destiné à renforcer la sécurité d'un procédé selon l'invention, le virus auxiliaire et le vecteur viral recombinant sont défectifs et peuvent se complémenter réciproquement, en totalité ou en partie. Une variante intéressante consiste à employer (i) un adénovirus auxiliaire selon l'invention défectif pour les fonctions E1 et E4 et comprenant les séquences loxP en 5' et en 3' de la région d'encapsidation, (atténuée ou non) (ii) un vecteur recombinant défectif pour toutes les fonctions à l'exception de E4 et (iii) une lignée cellulaire 293 produisant la recombinase hybride CRE-ER. Selon une autre alternative avantageuse, un procédé selon l'invention met en oeuvre (i) un adénovirus auxiliaire selon l'invention défectif pour les fonctions E1 et E4, comprenant les séquences loxP en 5' et en 3' de la région d'encapsidation (atténuée ou non) et produisant la recombinase hybride CRE-ER,(ii) un vecteur recombinant défectif pour toutes les fonctions à l'exception de E4 et (iii) une lignée cellulaire conventionnelle 293.

Les particules virales sont récupérées de la culture cellulaire, du milieu ou après lyse des cellules. Avantageusement, un procédé selon l'invention comprend une étape supplémentaire de purification des particules de vecteur viral recombinant. Bien que le choix de la technique soit large et à la portée de l'homme du métier, on peut citer plus particulièrement l'ultracentrifugation sur gradient de chlorure de césium ou de sucrose.

Enfin, l'invention vise également un procédé pour préparer une particule virale comprenant un vecteur viral recombinant par le moyen d'un virus auxiliaire, selon lequel le rapport particules virales de vecteur adénoviral recombinant sur celles de virus auxiliaire est supérieur à 50%, avantageusement supérieur à 60%, de préférence, supérieur à 70% et, de manière tout à fait préférée, supérieur à 80%.

L'invention a également trait à une particule de vecteur viral recombinant obtenue par un procédé selon l'invention ainsi qu'à une cellule eucaryote hôte selon l'invention. Ladite cellule hôte est avantageusement une cellule de mammifère et, de préférence, une cellule humaine et peut comprendre ledit vecteur sous forme intégrée dans le génome ou non intégrée (épisome). Il peut s'agir d'une cellule primaire ou tumorale d'une origine hématopoïétique (cellule souche totipotente, leucocyte, lymphocyte, monocyte ou macrophage...), musculaire, pulmonaire, trachéale, hépatique, épithéliale ou fibroblaste.

L'invention a également pour objet une composition pharmaceutique comprenant à titre d'agent thérapeutique ou prophylactique, une particule de vecteur viral recombinant obtenue par un procédé selon l'invention ou une cellule eucaryote hôte selon l'invention, en association avec un support acceptable d'un point de vue pharmaceutique. La composition selon l'invention est en particulier, destinée au traitement préventif ou curatif de maladies telles que :
- des maladies génétiques (hémophilie, mucoviscidose, diabète ou myopathie, celle de Duchêne et de Becker...),
- des cancers, comme ceux induits par des oncogènes ou des virus,
- des maladies virales, comme l'hépatite B ou C et le SIDA (syndrome de l'immunodéficience acquise résultant de l'infection par le VIH), et
- des maladies virales récurrentes, comme les infections virales provoquées par le virus de l'herpès.

Une composition pharmaceutique selon l'invention peut être fabriquée de manière conventionnelle. En particulier, on associe une quantité thérapeutiquement efficace d'un agent thérapeutique ou prophylactique à un support tel qu'un diluant. Une composition selon l'invention peut être administrée par aérosol, par voie locale ou encore systémique. Les voies d'administration envisageables dans le cadre de la présente invention peuvent être intragastrique, sous-cutanée, intracardiaque, intramusculaire, intraveineuse, intrapéritonéale, intratumorale, intrapulmonaire, nasale ou intratrachéale. L'administration peut avoir lieu en dose unique ou répétée une ou plusieurs fois après un certain délai d'intervalle. La voie d'administration et le dosage appropriés varient en fonction de divers paramètres, par exemple, de l'individu ou de la maladie à traiter ou encore du ou des gène(s) recombinant(s) à transférer. En particulier, les particules virales selon l'invention peuvent être formulées sous forme de doses comprises entre 10⁴ et 10¹⁴ ufp (unités formant des plages), avantageusement 10⁵ et 10¹³ ufp et, de préférence, 10⁶ et 10¹¹ ufp. La formulation peut également inclure un adjuvant acceptable d'un point de vue pharmaceutique.

Enfin, la présente invention est relative à l'usage thérapeutique ou prophylactique d'une particule de vecteur viral recombinant obtenue par un procédé selon l'invention ou d'une cellule eucaryote hôte selon l'invention pour la préparation d'un médicament destiné au traitement du corps humain ou animal et, préférentiellement, par thérapie génique. Selon une première possibilité, le médicament peut être administré directement *in vivo* (par exemple dans une tumeur accessible, dans les poumons par aérosol...). On peut également adopter l'approche *ex vivo* qui consiste à prélever des cellules du patient (cellules souches de la moëlle osseuse, lymphocytes du sang périphérique, cellules musculaires...), de les transfecter ou infecter *in vitro* selon les techniques de l'art et de les réadminister au patient.

L'invention s'étend également à une méthode de traitement selon laquelle on administre une quantité thérapeutiquement efficace d'une particule de vecteur viral recombinant obtenue par un procédé selon l'invention ou d'une cellule eucaryote hôte selon l'invention à un patient ayant besoin d'un tel traitement.

La présente invention est plus complètement décrite en référence aux Figures suivantes et à l'aide des exemples suivants.

La Figure 1 est une représentation schématique du génome de l'adénovirus humain de type 5 (représenté en unités arbitraires de 0 à 100), indiquant l'emplacement des différents gènes.

La Figure 2 est une représentation schématique du vecteur pTG4701 dans lequel la région 5' gauche de l'adénovirus 5 est modifié par l'insertion de répétitions directes LoxP de part et d'autre (en position 161 et 460) de la région d'encapsidation (psi).

La Figure 3 est une représentation schématique du vecteur pTG8595 délété des régions E1, E3 et E4 et comportant une cassette d'expression du gène LacZ contrôlé par le promoteur MLP et une séquence polyA (pA).

La Figure 4 est une représentation schématique du vecteur pTG4707 délété des régions E1, E3 et E4 et comprenant deux répétitions directes LoxP de part et d'autre de la région d'encapsidation psi.

La Figure 5 est une représentation schématique du vecteur pTG4662, vecteur adénoviral recombinant délété des régions E1 et E3 et portant la même cassette LacZ que pTG8595.

La Figure 6 est une représentation schématique du vecteur pTG4667 vecteur adénoviral recombinant délété des régions E1, E3 et des ORFs 1 à 4 de E4 et portant la même cassette LacZ que pTG8595.

La Figure 7 est une représentation schématique du vecteur pTG4702 qui dérive du vecteur précédent par insertion d'une cassette d'expression de la protéine hybride CRE-ER (indiquée cre et hER) à la place de celle de LacZ.

### EXEMPLES

Les exemples suivants n'illustrent qu'un mode de réalisation de la présente invention.

Les constructions décrites ci-dessous sont réalisées selon les techniques générales de génie génétique et de clonage moléculaire, détaillées dans Maniatis et al., (1989, Laboratory Manual, Cold Spring Harbor, Laboratory Press, Cold Spring Harbor, NY) ou selon les recommandations du fabricant lorsqu'on utilise un kit commercial. Les étapes de clonage mettant en oeuvre des plasmides bactériens sont réalisées dans la souche *Escherichia coli (E. coli)* 5K (Hubacek et Glover, 1970, J. Mol. Biol. *50,* 111-127) ou BJ5183 (Hanahan, 1983, J. Mol. Biol. *166*, 557-580). On utilise préférentiellement cette dernière souche pour les étapes de recombinaison homologue. Les techniques d'amplification par PCR sont connues de l'homme de l'art (voir par exemple PCR Protocols-A guide to methods and applications, 1990, edité par Innis, Gelfand, Sninsky et White, Academic Press Inc). S'agissant de la réparation des sites de restriction, la technique employée consiste en un remplissage des extrémités 5' protubérantes à l'aide du grand fragment de l'ADN polymérase I *d'E. coli* (Klenow).

En ce qui concerne la biologie cellulaire, les cellules sont transfectées selon les techniques standards bien connues de l'homme du métier. On peut citer la technique au phosphate de calcium (Maniatis et al., *supra*), mais tout autre protocole peut également être employé, tel que la technique au DEAE dextran, l'électroporation, les méthodes basées sur les chocs osmotiques, la microinjection d'une cellule sélectionnée ou les méthodes basées sur l'emploi de liposomes. Quant aux conditions de culture, elles sont classiques sauf lorsque spécifié.

Dans les exemples qui suivent, on a recours aux lignées cellulaires suivantes :
Lignée 293 dérivée de rein embryonnaire humain (Graham et al, 1977, *supra*) qui résulte de l'intégration dans ses chromosomes de l'extrémité 5' du génome de l'Ad5 (ITR5', séquence d'encapsidation et région E1) (disponible à l'ATCC sous la référence 1573).
Lignée TG1653 (décrite dans la demande internationale WO94/28152, exemple 8) qui dérive de la lignée 293 transformée de manière stable par le plasmide pTG1653 portant la région E4 de l'Ad5 (nt 32800 à 35826) et la cassette d'expression du gène *pac* (Puromycine Acétyl-Transférase) de résistance à la puromycine (Morgenstern et Land, 1990, Nucleic Acids Res. *18*, 3587-3596).

Il est entendu que d'autres lignées cellulaires peuvent être utilisées.

Par ailleurs, les fragments de génome adénoviral employés dans les différentes constructions décrites ci-après, sont indiqués précisément selon leurs positions dans la séquence nucléotidique du génome de l'Ad5 telle que divulguée dans la banque de données Genebank sous la référence M73260.

### EXEMPLE 1 : Constitution d'un virus auxiliaire.

Cet exemple décrit la construction d'un virus auxiliaire adénoviral défectif pour les fonctions E1 et E4 et comprenant une séquence de recombinaison loxP positionnée de part et d'autre de la région d'encapsidation.

La séquence loxP est portée par les oligonucléotides oTG6374 et oTG6522 (SEQ ID NO: 4 et 5). Ceux-ci sont réassociés puis introduits dans le site *Hin*dIII du vecteur p poly II (Lathe et al., Gene 57, 193-201), pour donner pTG4691.

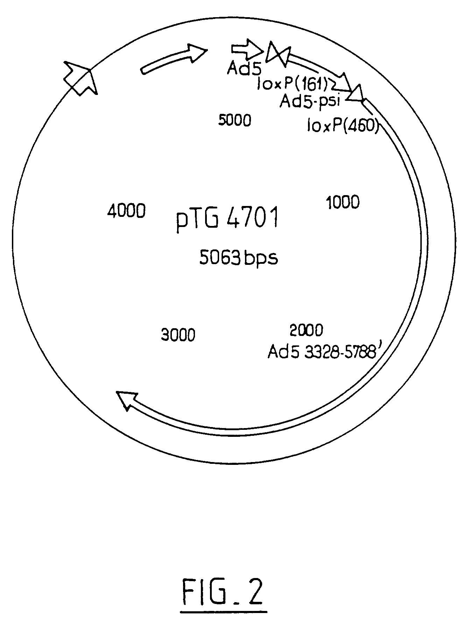

Le vecteur pTG8343 provient de l'insertion dans le vecteur p poly II d'une partie de l'extrémité 5' du génome adénoviral, à savoir les séquences s'étendant des nucléotides (nt) 1 à 458 et 3328 à 5788. Celui-ci est linéarisé par *Sal*I et mis en ligation avec le fragment *Sal*I-*Xho*I isolé de pTG4691 portant la séquence loxP. On obtient pTG4695 qui comprend une séquence loxP en position 450 du génome adénoviral soit en 3' de la région d'encapsidation. On clone une seconde séquence loxP sous forme d'un fragment *Sma*I-*Pvu*II purifié de pTG4691 dans le vecteur précédent linéarisé par *Afl*II (position 161) et traité à la Klenow. Le vecteur pTG4701 ainsi obtenu comporte deux répétitions directes loxP encadrant la région d'encapsidation psi (Figure 2).

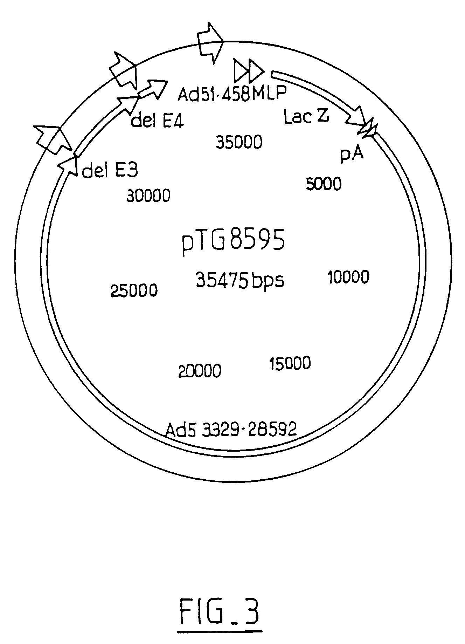

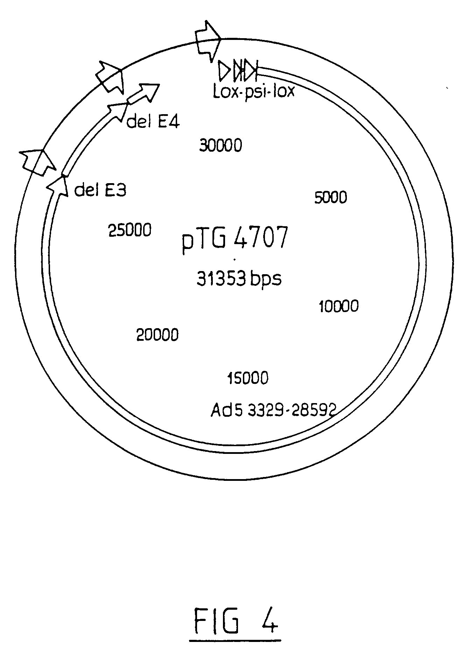

La région d'encapsidation modifiée est échangée contre son homologue génomique par la technique de recombinaison homologue. A cet effet, la souche *E. coli* est co-transformée avec le fragment *Bg*/I de pTG4701 et le vecteur pTG8595 (Figure 3) digéré par *Cla*I. Ce dernier est un vecteur adénoviral recombinant délété des régions E1 (nt 459-3328), E3 (nt 28592-30470) et E4 (nt 32800-35826) et comportant une cassette d'expression du gène LacZ sous le contrôle du promoteur MLP à la place de la région E1. Le vecteur recombiné est désigné pTG4707 (Figure 4). Après transfection dans la lignée de complémentation TG1653 (E1⁺, E4⁺) et amplification, les virus Ad TG4707 sont purifiés sur gradient de chlorure de césium afin de constituer un stock viral titrant environ 2 x 10⁸ pfu/ml.

### EXEMPLE 2 : Construction d'un vecteur adénoviral recombinant produisant une recombinase.

Cet exemple décrit la construction d'un vecteur adénoviral exprimant la protéine hybride CRE-ER. En absence d'oestrogène, cette dernière est produite sous une forme inactive mais en présence de l'hormone, elle adopte une conformation active. Le vecteur adénoviral est dépourvu de l'essentiel des régions E1 et E3 et de la partie de la région E4 autre que les ORFs 6 et 7 (Cadre de Lecture Ouvert pour Open Reading Frame en anglais). L'expression de ces deux gènes est suffisante pour assurer la fonction E4 sans nécessité de complémentation (Ketner et al., 1989, Nucleic Acids Res. *17*, 3037-3048). Deux types de vecteurs ont été construits. Dans le premier (pTG4708), le gène CRE-ER est placé sous le contrôle du promoteur SV40 alors que dans le second (pTG5630), il est dirigé par le promoteur CMV.

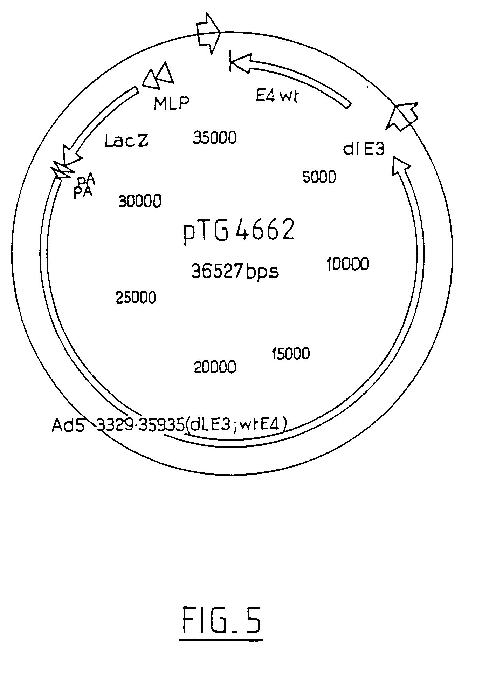

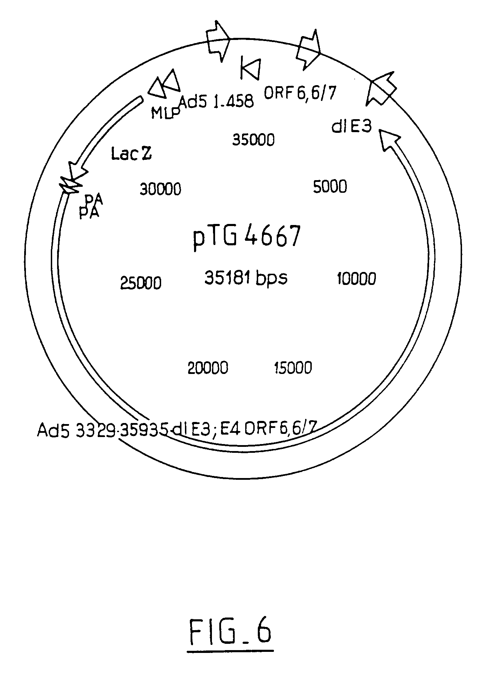

En premier lieu, le vecteur pTG1653 portant la région E4 de l'Ad5 (nt 32800 à 35826 ; voir WO 94/28152), est digéré par *Avr*II et *Bgl*II puis traité par la Klenow avant d'être religué sur lui-même. On obtient pTG4660 qui porte une cassette d'expression des ORFs 6 et 7 sous le contrôle du promoteur homologue E4. Celle-ci est introduite dans un vecteur adénoviral par recombinaison homologue. A cet effet, on choisit le vecteur pTG4662 (Figure 5) qui comporte l'ITR 5' et les séquences d'encapsidation (nt 1 à 458), une cassette d'expression du gène LacZ à la place de la région E1 et les séquences adénovirales restantes délétées de la région E3 (nt 3329 à 27870 et 30749 à 35935). La souche *E. coli* BJ est co-transformée par le fragment *Fsp*I- *Mun*I issu de pTG4660 et pTG4662 digéré par *Swa*I. Cet événement de recombinaison permet de générer pTG4667 (Figure 6) dans lequel la cassette ORFs 6 et 7 remplace la région sauvage E4.

### A. Construction du vecteur pTG4708

En parallèle, la cassette d'expression de la recombinase hybride CRE-ER est isolée du vecteur pCre-ER (Metzger et al., 1995, *supra*) sous forme d'un fragment *Sal*I et clonée dans le vecteur pTG8343 préalablement linéarisé par cette même enzyme. On obtient pTG4699 et pTG4700 qui diffèrent par l'orientation de la cassette.

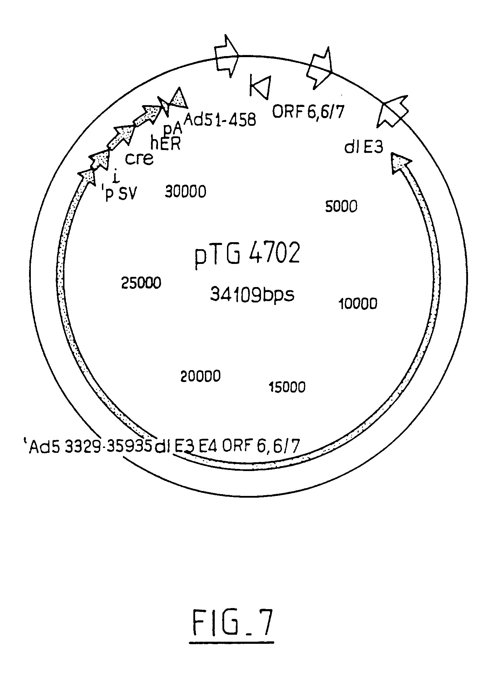

Les vecteurs pTG4702 (Figure 7) et pTG4703 sont obtenus par recombinaison homologue entre un fragment *Sgr*AI-*Bst*EII purifié de pTG4699 et pTG4700 respectivement et le vecteur adénoviral pTG4667 linéarisé par *Cla*I. Ils portent la cassette d'expression CRE-ER (orientation différente pour chacun d'entre eux) à la place de la région E1 et sont dépourvus de la région E3 et des ORFs 1 à 4 de E4. Ils sont transfectés dans les cellules 293 et 1653 afin de s'assurer que l'expression de la recombinase n'est pas toxique à la croissance cellulaire et à la multiplication virale.

La construction d'un vecteur adénoviral, en outre, défectif pour la région E2 peut être réalisée à partir des constructions précédentes par traitement par l'enzyme *Asc*I et religation. On isole les clones qui ont réintégré le fragment *Asc*I mais en orientation opposée par rapport au vecteur parental. Les candidats peuvent être déterminés par simple digestion enzymatique avec des enzymes dont les sites sont présents sur le fragment, par exemple *Bam*HI et sont désignés pTG4708. Cette inversion de l'orientation a pour but d'interrompre les unités de transcription codant pour E2A, la polymérase et l'hexon et de rendre la fonction E2 défective. Il est également possible d'introduire des délétions de tout ou partie de la région E2.

### B. Construction du vecteur pTG5630

Le vecteur pTG4667 est digéré par l'enzyme *Sna*BI puis religué. On selectionne le vecteur pTG5613 similaire à pTG4667 mis à part l'inversion du fragment adénoviral *Sna*BI (positions 10307 à 25173) recouvrant une grande partie des séquences codantes de la région E2. Cette inversion permet de générer un virus défectif incapable de produire les produits d'expression fonctionnels de E2.

Par ailleurs, les séquences codant pour CRE-ER sont isolées par digestion *Eco*RI et traitées par la Klenow avant d'être introduites en aval du promoteur CMV, donnant lieu à pTG5625. On obtient le vecteur pTG5630 par recombinaison homologue entre le fragment *Pac*I-*Bst*EII isolé de pTG5625 et le vecteur pTG5617 linéarisé par *Cla*I. pTG5630 est défectif pour les fonctions E1 et E2, déleté de la majeure partie de E3, et porte la cassette "promoteur CMV - CRE-ER" à la place de E1 en orientation inverse par rapport à l'ITR 5'.

### EXEMPLE 3 : Procédé de préparation de particules virales d'un vecteur adénoviral par le système CRE et loxP.

### A. Mise en oeuvre du vecteur pTG4708 et de l'auxiliaire pTG4707

Dans cet exemple, on utilise :
(1) un vecteur adénoviral (pTG4708) défectif pour l'ensemble des fonctions adénovirales à l'exception de E4 et portant les séquences codant pour la recombinase CRE-ER,
(2) un virus auxiliaire (pTG4707) défectif pour les fonctions E1, E3 et E4 et contenant deux répétitions directes loxP placées de part et d'autre de la région d'encapsidation, et
(3) la lignée cellulaire de complémentation 293 complémentant la fonction E1.

Les vecteurs pTG4707 et pTG4708 sont co-transfectés dans les cellules 293 et cultivées dans un premier temps dans un milieu classique ne contenant pas d'oestrogène. Les particules virales peuvent être formées dans les cellules qui comportent les deux vecteurs puisqu'ils se complémentent mutuellement. En effet, les protéines E2 sont produites à partir du virus auxiliaire, les protéines E4 à partir de vecteur adénoviral et les protéines E1 sont fournies par la lignée cellulaire. Par ailleurs, la protéine hybride CRE-ER est produite sous sa forme inactive puisque le milieu de culture n'est pas supplémenté en hormone de sorte que le génome du virus auxiliaire est apte à être encapsidé. Les plages ainsi produites contiennent une population mixte de virus, une partie contenant le génome du vecteur adénoviral et l'autre partie celui du virus auxiliaire. Cette étape permet une amplification de la quantité de virus dans le but d'améliorer les titres.

L'étape de production sélective des particules de vecteur adénoviral est réalisée en introduisant dans le milieu de culture de l'estradiol selon les conditions détaillées dans Metzger et al. (1995, *supra*). La présence d'estradiol va permettre d'activer la recombinase CRE-ER qui, une fois fonctionnelle, est apte à induire un événement de recombinaison entre les séquences loxP. Les virus générés dans ces conditions sont purifiés, l'ADN est isolé et la délétion de la région d'encapsidation vérifiée soit par digestion enzymatique ou hybridation à une sonde appropriée complémentaire à celle-ci selon la technologie de Southern.

### B. Mise en oeuvre du vecteur pTG5630 et de l'auxiliaire pTG4707

2,5 à 5 µg de vecteur pTG5630 sont transfectés dans la lignée 293 dans les conditions classiques. Le lendemain les cellules transfectées sont surinfectées par le virus auxiliaire AdTG4707 à raison d'environ 0,04 pfu/cellule et cultivées dans un milieu DMEM depleté en rouge de phenol (ce dernier étant susceptible de présenter une activité oestrogène). Deux conditions de culture ont été étudiées en parallèle : milieu supplémenté en β- oestradiol (Sigma ; E4389) à une concentration de 10⁻⁶ et 10⁻⁷ M après le troisième passage et milieu non supplémenté. La culture cellulaire est récoltée à 4 jours post-infection et une partie de la récolte amplifiée par passages successifs sur des cellules 293 fraîches. L'autre partie est conservée pour les analyses d'ADN viral. On observe une différence significative de l'état des cellules selon les conditions de culture. En effet, la cytophathie est moins prononcée en présence d'oestradiol qu'en son absence, ceci à compter du troisième passage, ce qui laisse présager une différence au niveau de la production virale.

Afin de vérifier ce point, l'ADN des virus produits lors des quatre premiers passages est isolé d'un même volume de culture, digéré par l'enzyme *Mun*I et analysé par Southern à l'aide d'une sonde radioactive complémentaire à une séquence en amont de la région E4. Après hybridation, on visualise un fragment *Mun*I de 1,7 kb dans le cas des virus issus du vecteur pTG5630 et un fragment *Mun*I de 1,1 kb dans le cas des virus auxiliaires AdTG4707. Ces conditions permettent d'apprécier la quantité relative de virus d'intérêt et auxiliaires générés à chaque cycle d'amplification.

Dans les conditions où la recombinase CRE-ER est inactive (en absence d'oestrogène), on observe une amplification simultanée des deux types de virus se traduisant par un signal de plus en plus intense au fur et à mesure des passages. En revanche, lorsque le milieu de culture est supplémenté en oestradiol, le signal correspondant au virus auxiliaire diminue alors que celui spécifique pour AdTG5630 augmente à chaque passage. Ces résultats indiquent que l'activation de la recombinase s'accompagne d'une amplification préférentielle du virus d'intérêt.

L'excision de la région d'encapsidation du virus auxiliaire est mise en évidence par analyse Southern sur les préparations d'ADN viral digéré par l'enzyme *Afl*II et en mettant en oeuvre une sonde spécifique de la région d'encapsidation s'hybridant à un fragment de 3,7 kb dans le cas du virus AdTG5630 et à un fragment de soit 800 pb dans le cas du virus auxiliaire intègre ou 400 pb dans le cas où les séquences d'encapsidation bordées par les sites *lox*P sont excisées.

L'intensité du signal correspondant au fragment de 3,7 kb augmente au fur et à mesure des cycles d'amplification quelles que soient les conditions de culture alors que celui correspondant au fragment 800 pb ne croît qu'en absence d'oestradiol. Par contre, en présence de l'hormone, le signal correspondant au virus auxiliaire s'affaiblit. A forte exposition, on peut mettre en évidence une bande à 400 pb indiquant que la région d'encapsidation des virus auxiliaires est excisée par l'action de la recombinase.

Dans leur ensemble, ces résultats montrent que le système CRE-LoxP peut être adapté aux adénovirus pour réduire la contamination des préparations adénovirales par le virus auxiliaire.

### EXEMPLE 4 : Constitution d'une lignée stable exprimant la recombinase hybride CRE-ER.

Le vecteur pCre-ER est transfecté, de manière conventionnelle, dans les cellules 293 en même temps qu'un vecteur de sélection (par exemple pRC-CMV conférant la résistance à la néomycine disponible commercialement (Invitrogen), pTG1643 conférant la résistance à la puromycine décrit dans WO 94/28156). Après transfection, les cellules sont cultivées en milieu sélectif contenant l'antibiotique et on isole les clones résistants (désignés 293/CRE-ER) qui sont testés pour leur capacité à produire un vecteur adénoviral.

On agit selon la même technologie pour générer une lignée 1653 (complémentant les fonctions adénovirales E1 et E4) exprimant le produit du gène CRE-ER.

Les cellules 293/CRE-ER ainsi générées sont co-transfectées par le virus auxiliaire pTG4707 et un vecteur adénoviral recombinant défectif pour toutes les fonctions à l'exception de E4 (tels que ceux décrits dans WO 94/28152) puis cultivées dans un milieu classique de manière à générer une population mixte et amplifiées de particules virales. Après un certain temps, le milieu de culture est remplacé par un milieu contenant de l'oestradiol afin de produire la recombinase sous sa forme active et empêcher la production de particules virales AdTG4707.

La lignée 1653/CRE-ER sera utile pour la préparation de vecteurs défectifs pour l'ensemble des fonctions essentielles (voir WO 94/28156). La technologie employée est comparable à celle décrite précédemment à savoir : co-transfection par le virus auxiliaire pTG4707 et un vecteur adénoviral recombinant minimum, culture en milieu sélectif non supplémenté en oestradiol pendant un temps suffisant puis ajout de l'hormone dans le milieu de culture et récupération des particules virales produites.

## Revendications

1. Lignée cellulaire de complémentation pour la production d'un vecteur viral défectif pour la réplication comprenant :
(i) un premier fragment d'ADN capable de complémenter *en trans* les fonctions défectives d'un tel vecteur, et
(ii) un second fragment d'ADN codant pour une protéine hybride résultant de la fusion d'un polypeptide ayant une activité recombinase et d'un domaine de liaison à un ligand hormonal et capable de reconnaître une séquence de recombinaison.

2. Lignée cellulaire de complémentation selon la revendication 1, **caractérisée en ce que** ladite recombinase est inactive en absence dudit ligand.

3. Lignée cellulaire de complémentation selon l'une des revendications 1 et 2, **caractérisée en ce que** ledit polypeptide ayant une activité recombinase est la recombinase CRE.

4. Lignée cellulaire de complémentation selon l'une des revendications 1 à 3, **caractérisée en ce que** ledit domaine de liaison est le domaine de liaison au ligand du récepteur oestrogène humain ER.

5. Lignée cellulaire de complémentation selon l'une des revendications 1 à 4, **caractérisée en ce qu'**elle est capable de complémenter la fonction E1 et/ou E4 d'un vecteur adénoviral défectif pour la réplication.

6. Lignée cellulaire de complémentation selon l'une des revendications 1 à 5, **caractérisée en ce qu'**elle est générée à partir de la lignée 293.

7. Virus auxiliaire pour la production d'un vecteur viral recombinant défectif pour la réplication, comprenant :
(i) une première et une seconde séquence de recombinaison localisées en 5' et en 3' d'une région essentielle à la propagation dudit virus auxiliaire, lesdites séquences de recombinaison étant reconnues par une recombinase, et
(ii) une cassette d'expression codant pour une recombinase qui est une protéine hybride résultant de la fusion d'un polypeptide ayant une activité recombinase et d'un domaine de liaison à un ligand hormonal.

8. Virus auxiliaire selon la revendication 7, **caractérisé en ce que** ladite recombinase est inactive en absence dudit ligand.

9. Virus auxiliaire selon l'une des revendications 7 et 8, **caractérisé en ce que** ledit polypeptide ayant une activité recombinase est la recombinase CRE.

10. Virus auxiliaire selon l'une des revendications 7 à 9, **caractérisé en ce que** ledit domaine de liaison est le domaine de liaison aux ligands du récepteur oestrogène humain ER.

11. Virus auxiliaire selon l'une des revendications 7 à 10, **caractérisé en ce que** ladite région essentielle à la propagation dudit virus est sélectionnée dans le groupe comprenant des éléments nécessaires à l'expression d'un gène et des éléments agissant en cis.

12. Virus auxiliaire selon l'une des revendications 7 à 11, **caractérisé en ce qu'**il comprend un ou plusieurs gène(s) essentiel(s) à la replication virale qui est(sont) inactif(s).

13. Virus auxiliaire selon l'une des revendications 7 à 12, **caractérisé en ce que** lesdites séquences de recombinaison sont positionnées en 5' et 3' de la région d'encapsidation dudit virus.

14. Virus auxiliaire selon l'une des revendications 7 à 13, **caractérisé en ce que** lesdites première et seconde séquences de recombinaison sont positionnées dans la même orientation l'une par rapport à l'autre.

15. Virus auxiliaire selon l'une des revendications 7 à 14, **caractérisé en ce que** lesdites première et seconde séquences de recombinaison sont sélectionnées parmi le groupe constitué par les séquences loxP, FRT et R.

16. Virus auxiliaire selon l'une des revendications 7 à 15, pour la production d'un vecteur adénoviral recombinant défectif pour la réplication, qui dérive du génome d'un adénovirus et comprenant :
(i) les ITRs 5' et 3', une région d'encapsidation et au moins un gène viral sélectionné parmi les gènes des régions E1, E2, E4 et L1-L5, ledit gène étant défectif dans ledit vecteur adénoviral recombinant,
(ii) une première et une seconde séquences de recombinaison localisées en 5' et en 3' de ladite région d'encapsidation.

17. Virus auxiliaire selon la revendication 16, **caractérisé en ce qu'**il est dépourvu de tout ou partie de la région E1 et/ou E4.

18. Procédé pour préparer une particule virale comprenant un vecteur viral recombinant, qui comprend les étapes suivantes :
a) préparer un vecteur viral recombinant déficient pour la replication ;
b) préparer un virus auxiliaire selon l'une des revendications 7 à 17 ;
c) introduire le vecteur viral recombinant et le virus auxiliaire dans une lignée cellulaire appropriée ;
d) cultiver ladite lignée cellulaire dans des conditions appropriées pour permettre la production de la particule virale en présence d'une recombinase fonctionnelle capable de reconnaître lesdites première et seconde séquences de recombinaison ; et
e) récupérer la particule virale dans la culture cellulaire.

19. Procédé selon la revendication 18, **caractérisé en ce que** ladite recombinase est une protéine hybride CRE-ER, lesdites première et seconde séquences de recombinaison sont des séquences loxP et ledit ligand est l'oestradiol.

20. Procédé selon l'une des revendications 18 et 19, **caractérisé en ce que** ladite lignée cellulaire est la lignée cellulaire de complémentation 293.

21. Procédé selon la revendication 20, qui comprend les étapes suivantes :
a) préparer un vecteur adénoviral recombinant défectif pour l'ensemble des fonctions adénovirales à l'exception de E4 ;
b) préparer un virus auxiliaire défectif pour les fonctions E1 et E4 et comprenant (i) les séquences loxP en 5' et en 3' de la région d'encapsidation et (ii) un fragment d'ADN codant pour la recombinase hybride CRE-ER ;
c) introduire le vecteur viral recombinant et le virus auxiliaire dans une lignée cellulaire 293 ;
d) cultiver ladite lignée cellulaire dans des conditions appropriées pour permettre la production de la particule virale et, après un temps suffisamment long, poursuivre la culture dans un milieu comprenant de l'oestradiol ; et
e) récupérer la particule virale dans la culture cellulaire.

22. Procédé selon la revendication 18, **caractérisé en ce que** ledit virus auxiliaire est défini selon l'une des revendications 7 à 17 et **en ce que** les cellules de complémentation sont définies selon l'une des revendications 1 à 6.

23. Procédé selon la revendication 20, qui comprend les étapes suivantes :
a) préparer un vecteur adénoviral recombinant défectif pour l'ensemble des fonctions adénovirales à l'exception de E4 ;
b) préparer un virus auxilaire défectif pour les fonctions E1 et E4 et comprenant les séquences loxP en 5' et 3' de la région d'encapsidation ;
c) introduire le vecteur viral recombinant et le virus auxiliaire dans une lignée cellulaire 293 comprenant un fragment d'ADN codant pour la recombinase hybride CRE-ER ;
d) cultiver ladite lignée cellulaire dans des conditions appropriées pour permettre la production de la particule virale et, après un temps suffisamment long, poursuivre la procédure dans un milieu comprenant l'oestradiol ; et
e) récupérer la particule virale dans la culture cellulaire.

24. Procédé selon l'une des revendications 18 à 23, qui comprend en outre une étape de purification du vecteur viral recombinant.

25. Lignée cellulaire de complémentation renfermant un virus auxiliaire selon l'une des revendications 7 à 17.

## Claims

1. Complementation cell line for the production of a viral vector defective for replication comprising:
(i) a first DNA fragment capable of complementing *in trans* the defective functions of such a vector, and
(ii) a second DNA fragment coding for a hybrid protein resulting from the fusion of a polypeptide having a recombinase activity and a region of linkage to a hormone ligand and capable of recognizing a recombination sequence.

2. Complementation cell line according to Claim 1, **characterized in that** the said recombinase is inactive in the absence of the said ligand.

3. Complementation cell line according to either of Claims 1 and 2, **characterized in that** the said polypeptide having a recombinase activity is the recombinase CRE.

4. Complementation cell line according to one of Claims 1 to 3, **characterized in that** the said region of linkage is the region of linkage to the ligand of the human oestrogen receptor ER.

5. Complementation cell line according to one of Claims 1 to 4, **characterized in that** it is capable of complementing the function E1 and/or E4 of an adenoviral vector defective for replication.

6. Complementation cell line according to one of Claims 1 to 5, **characterized in that** it is generated from the line 293.

7. Helper virus for the production of a recombinant viral vector defective for replication, comprising:
(i) a first and a second recombination sequence located at 5' and at 3' of a region essential for the propagation of the said helper virus, the said recombination sequences being recognized by a recombinase, and
(ii) an expression cassette coding for a recombinase which is a hybrid protein resulting from the fusion of a polypeptide having a recombinase activity and a linkage region to a hormone ligand.

8. Helper virus according to Claim 7, **characterized in that** the said recombinase is inactive in the absence of the said ligand.

9. Helper virus according to either of Claims 7 and 8, **characterized in that** the said polypeptide having a recombinase activity is the recombinase CRE.

10. Helper virus according to one of Claims 7 to 9, **characterized in that** the said region of linkage is the region of linkage to the ligands of the human oestrogen receptor ER.

11. Helper virus according to one of Claims 7 to 10, **characterized in that** the said region essential to the propagation of the said virus is selected from the comprising elements necessary for the expression of a gene and of elements acting in cis.

12. Helper virus according to one of Claims 7 to 11, **characterized in that** it comprises one or more gene(s) essential for viral replication which is(are) inactive.

13. Helper virus according to one of Claims 7 to 12, **characterized in that** the said recombination sequences are positioned at 5' and 3' of the encapsidation region of the said virus.

14. Helper virus according to one of Claims 7 to 14, **characterized in that** the said first and second recombination sequences are positioned in the same orientation with respect to one another.

15. Helper virus according to one of Claims 7 to 14, **characterized in that** the said first and second recombination sequences are selected from the group formed by the sequences loxP, FRT and R.

16. Helper virus according to one of Claims 7 to 15, for the production of a recombinant adenoviral vector defective for replication, which derives from the genome of an adenovirus and comprising:
(i) the ITRs 5' and 3', an encapsidation region and at least one viral gene selected from the genes of the E1, E2, E4 and L1-L5 regions, the said gene being defective in the said recombinant adenoviral vector,
(ii) a first and a second recombination sequence located at 5' and at 3' of the said encapsidation region.

17. Helper virus according to Claim 16, **characterized in that** it is devoid of all or part of the E1 and/or E4 region.

18. Procedure for preparing a viral particle comprising a recombinant viral vector, which comprises the following steps:
a) preparing a recombinant viral vector deficient for replication;
b) preparing a helper virus according to one of Claims 7 to 17;
c) introducing the recombinant viral vector and the helper virus into an appropriate cell line;
d) culturing the said cell line under appropriate conditions to allow the production of the viral particle in the presence of a functional recombinase able to recognize the said first and second recombination sequences; and
e) recovering the viral particle in the cell culture.

19. Procedure according to Claim 18, **characterized in that** the said recombinase is a hybrid protein CRE-ER, the said first and second recombination sequences are loxP sequences and the said ligand is oestradiol.

20. Procedure according to either of Claims 18 and 19, **characterized in that** the said cell line is the 293 complementation cell line.

21. Procedure according to Claim 20, which comprises the following steps:
a) preparing a recombinant adenoviral vector defective for all of the adenoviral functions with the exception of E4;
b) preparing a helper virus defective for the E1 and E4 functions and comprising (i) the LoxP sequences at 5' and at 3' of the encapsidation region and (ii) a DNA fragment coding for the CRE-ER hybrid recombinase;
c) introducing the recombinant viral vector and the helper virus into a 293 cell line;
d) culturing the said cell line under appropriate conditions to allow the production of the viral particle and, after a sufficiently long time, continuing the culture in a medium comprising oestradiol; and
e) recovering the viral particle in the cell culture.

22. Procedure according to Claim 18 **characterized in that** the said helper virus is defined according to one of Claims 7 to 17 and **in that** the complementation cells are defined according to one of Claims 1 to 6.

23. Procedure according to Claim 20, which comprises the following steps:
a) preparing a recombinant adenoviral vector defective for all of the adenoviral functions with the exception of E4;
b) preparing a helper virus defective for the E1 and E4 functions and comprising the LoxP sequences at 5' and 3' of the encapsidation region;
c) introducing the recombinant viral vector and the helper virus into a 293 cell line comprising a DNA fragment coding for the CRE-ER hybrid recombinase.
d) culturing said cell line under appropriate conditions to allow the production of the viral particle and, after a sufficiently long time, continuing the culture in a medium comprising oestradiol; and
e) recovering the viral particle in the cell culture.

24. Procedure according to one of Claims 18 to 23, which additionally comprises a step of purification of the recombinant viral vector.

25. Complementation cell line comprising a helper virus according to one of Claims 7 to 17.

## Patentansprüche

1. Komplementations-Zelllinie für die Produktion eines replikationsdefekten viralen Vektors, umfassend:
(i) ein erstes DNA-Fragment, das die defekten Funktionen eines solchen Vektors in trans komplementieren kann, und
(ii) ein zweites DNA-Fragment, das für ein Hybridprotein codiert, welches aus der Fusion eines Polypeptids mit einer Rekombinase-Aktivität und einer Domäne der Bindung an einen hormonellen Liganden resultiert und in der Lage ist, eine Rekombinationssequenz zu erkennen.

2. Komplementations-Zelllinie nach Anspruch 1, **dadurch gekennzeichnet, dass** die Rekombinase in Abwesenheit des Liganden inaktiv ist.

3. Komplementations-Zelllinie nach einem der Ansprüche 1 und 2, **dadurch gekennzeichnet, dass** das Polypeptid, das eine Rekombinase-Aktivität besitzt, die Rekombinase CRE ist.

4. Komplementations-Zelllinie nach einem der Ansprüche 1 bis 3, **dadurch gekennzeichnet, dass** die Domäne der Bindung die Domäne der Bindung an den Liganden des humanen Östrogen-Rezeptors ER ist.

5. Komplementations-Zelllinie nach einem der Ansprüche 1 bis 4, **dadurch gekennzeichnet, dass** sie in der Lage ist, die Funktion E1 und/oder E4 eines replikationsdefekten adenoviralen Vektors zu komplementieren.

6. Komplementations-Zelllinie nach einem der Ansprüche 1 bis 5, **dadurch gekennzeichnet, dass** sie ausgehend von der Linie 293 erzeugt wird.

7. Hilfsvirus für die Produktion eines rekombinanten replikationsdefekten viralen Vektors, umfassend:
(i) eine erste und eine zweite Rekombinationssequenz, die in 5' und in 3' von einer Region angeordnet sind, die für die Fortpflanzung des Hilfsvirus essentiell ist, wobei die Rekombinationssequenzen durch eine Rekombinase erkannt werden, und
(ii) eine Expressionskassette, die für eine Rekombinase codiert, welche ein Proteinhybrid ist, das aus der Fusion eines Polypeptids mit einer Rekombinase-Aktivität und einer Domäne der Bindung an einen hormonellen Liganden resultiert.

8. Hilfsvirus nach Anspruch 7, **dadurch gekennzeichnet, dass** die Rekombinase in Abwesenheit des Liganden inaktiv ist.

9. Hilfsvirus nach einem der Ansprüche 7 und 8, **dadurch gekennzeichnet, dass** das Polypeptid, das eine Rekombinase-Aktivität besitzt, die Rekombinase CRE ist.

10. Hilfsvirus nach einem der Ansprüche 7 bis 9, **dadurch gekennzeichnet, dass** die Domäne der Bindung die Domäne der Bindung an die Liganden des humanen Östrogen-Rezeptors ER ist.

11. Hilfsvirus nach einem der Ansprüche 7 bis 10, **dadurch gekennzeichnet, dass** die Region, die für die Fortpflanzung des Virus essentiell ist, aus der Gruppe ausgewählt ist, welche Elemente, die zur Expression eines Gens notwendig sind, und Elemente, die in cis aktiv sind, umfasst.

12. Hilfsvirus nach einem der Ansprüche 7 bis 11, **dadurch gekennzeichnet, dass** es ein oder mehrere für die virale Replikation wesentliche(s) Gen(e) umfasst, das bzw. die inaktiv ist (sind).

13. Hilfsvirus nach einem der Ansprüche 7 bis 12, **dadurch gekennzeichnet, dass** die Rekombinationssequenzen in 5' und 3' der Verpackungsregion des Virus angeordnet sind.

14. Hilfsvirus nach einem der Ansprüche 7 bis 13, **dadurch gekennzeichnet, dass** die erste und zweite Rekombinationssequenz in der gleichen Orientierung bezüglich einander angeordnet sind.

15. Hilfsvirus nach einem der Ansprüche 7 bis 14, **dadurch gekennzeichnet, dass** die erste und zweite Rekombinationssequenz aus der Gruppe ausgewählt sind, die aus den Sequenzen loxP, FRT und R besteht.

16. Hilfsvirus nach einem der Ansprüche 7 bis 15 für die Produktion eines rekombinanten replikationsdefekten adenoviralen Vektors, der von dem Genom eines Adenovirus abstammt und umfasst:
(i) die ITRs 5' und 3', eine Verpackungsregion und mindestens ein virales Gen, das aus Genen der Regionen E1, E2, E4 und L1-L5 ausgewählt ist, wobei das Gen in dem rekombinanten adenoviralen Vektor defekt ist,
(ii) eine erste und eine zweite Rekombinationssequenz, die in 5' und in 3' der Verpackungsregion angeordnet ist.

17. Hilfsvirus nach Anspruch 16, **dadurch gekennzeichnet, dass** ihm die ganze oder ein Teil der Region E1 und/oder E4 fehlt.

18. Verfahren zur Herstellung eines viralen Partikels, das einen rekombinanten viralen Vektor umfasst, welches die folgenden Schritte umfasst:
a) Herstellen eines rekombinanten replikationsdefekten viralen Vektors;
b) Herstellen eines Hilfsvirus nach einem der Ansprüche 7 bis 17;
c) Einführen des rekombinanten viralen Vektors und des Hilfsvirus in eine geeignete Zelllinie;
d) Kultivieren der Zelllinie unter geeigneten Bedingungen, um die Produktion des viralen Partikels in Anwesenheit einer funktionellen Rekombinase zu gestatten, welche die erste und zweite Rekombinationssequenz erkennen kann; und
e) Gewinnen des viralen Partikels aus der Zellkultur.

19. Verfahren nach Anspruch 18, **dadurch gekennzeichnet, dass** die Rekombinase ein Proteinhybrid CRE-ER ist, die erste und zweite Rekombinationssequenz die Sequenzen loxP sind und der Ligand Östradiol ist.

20. Verfahren nach einem der Ansprüche 18 und 19, **dadurch gekennzeichnet, dass** die Zelllinie die Komplementations-Zelllinie 293 ist.

21. Verfahren nach Anspruch 20, welches die folgenden Schritte umfasst:
a) Herstellen eines rekombinanten adenoviralen Vektors, der bezüglich der Gesamtheit der adenoviralen Funktionen mit Ausnahme von E4 defekt ist;
b) Herstellen eines Hilfsvirus, der bezüglich der Funktionen E1 und E4 defekt ist und umfasst: (i) die Sequenzen loxP in 5' und in 3' der Verpackungsregion und (ii) ein DNA-Fragment, das für die Hybridrekombinase CRE-ER codiert;
c) Einführen des rekombinanten viralen Vektors und des Hilfsvirus in eine Zelllinie 293;
d) Kultivieren der Zelllinie unter Bedingungen, die geeignet sind, um die Produktion des viralen Partikels zu gestatten und nach einer ausreichend langen Zeit Fortsetzen der Kultur in einem Medium, das Östradiol umfasst; und
e) Gewinnen des viralen Partikels aus der Zellkultur.

22. Verfahren nach Anspruch 18, **dadurch gekennzeichnet, dass** das Hilfsvirus gemäß einem der Ansprüche 7 bis 10 definiert ist und dass die Komplementationszellen gemäß einem der Ansprüche 1 bis 6 definiert sind.

23. Verfahren nach Anspruch 20, welches die folgenden Schritte umfasst:
a) Herstellen eines rekombinanten adenoviralen Vektors, der bezüglich der Gesamtheit der adenoviralen Funktionen mit Ausnahme von E4 defekt ist;
b) Herstellen eines Hilfsvirus der bezüglich der Funktionen E1 und E4 defekt ist und die Sequenzen loxP in 5' und 3' der Verpackungsregion umfasst;
c) Einführen des rekombinanten viralen Vektors und des Hilfsvirus in eine Zelllinie 293, die ein DNA-Fragment umfasst, das für die Hybridrekombinase CRE-ER codiert;
d) Kultivieren der Zelllinie unter Bedingungen, die geeignet sind, die Produktion des viralen Partikels zu gestatten, und nach einer hinreichend langen Zeit Fortsetzen des Verfahrens in einem Medium, das Östradiol umfasst; und
e) Gewinnen des viralen Partikels aus der Zellkultur.

24. Verfahren nach einem der Ansprüche 18 bis 23, welches darüber hinaus einen Schritt der Reinigung des rekombinanten viralen Vektors umfasst.

25. Komplementations-Zelllinie, die einen Hilfsvirus nach einem der Ansprüche 7 bis 17 einschließt.


### LISTE DE SEQUENCES

(1) INFORMATION GENERALE:
   (i) DEPOSANT:
      (A) NOM: Transgene S.A.
      (B) RUE: 11, rue de Molsheim
      (C) VILLE: Strasbourg
      (E) PAYS: France
      (F) CODE POSTAL: 67082
      (G) TELEPHONE: (33) 88 27 91 00
      (H) TELECOPIE: (33) 88 27 91 11
   (ii) TITRE DE L' INVENTION: Nouveaux virus auxiliaires pour la préparation de vecteurs viraux recombinants.
   (iii) NOMBRE DE SEQUENCES: 5
   (iv) FORME LISIBLE PAR ORDINATEUR:
      (A) TYPE DE SUPPORT: Tape
      (B) ORDINATEUR: IBM PC compatible
      (C) SYSTEME D'EXPLOITATION: PC-DOS/MS-DOS
      (D) LOGICIEL: Patent In Release #1.0, Version #1.25 (OEB)
(2) INFORMATION POUR LA SEQ ID NO: 1:
   (i) CARACTERISTIQUES DE LA SEQUENCE:
      (A) LONGUEUR: 34 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: Bacteriophage P1
      (B) SOUCHE: loxP (locus de crossing over)
   (xi) DESCRIPTION DE LA SEQUENCE: SEQ ID NO: 1 :
   ATAACTTCGT ATAATGTATG CTATACGAAG TTAT 34
(2) INFORMATION POUR LA SEQ ID NO: 2:
   (i) CARACTERISTIQUES DE LA SEQUENCE:
      (A) LONGUEUR: 34 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: Saccharomyces cerevisiae
      (B) SOUCHE: FRT
   (xi) DESCRIPTION DE LA SEQUENCE: SEQ ID NO: 2:
   GAAGTTCCTA TACTTTCTAG AGAATAGGAA CTTC 34
(2) INFORMATION POUR LA SEQ ID NO: 3:
   (i) CARACTERISTIQUES DE LA SEQUENCE:
      (A) LONGUEUR: 31 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: Zygosaccharomyces rouxii
      (B) SOUCHE: Site R
   (xi) DESCRIPTION DE LA SEQUENCE: SEQ ID NO: 3:
   TTGATGAAAG AATACGTTAT TCTTTCATCA A                31
(2) INFORMATION POUR LA SEQ ID NO: 4:
   (i) CARACTERISTIQUES DE LA SEQUENCE:
      (A) LONGUEUR: 45 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: oligonucleotide de synthese (loxP)
      (B) SOUCHE: oTG6374
   (xi) DESCRIPTION DE LA SEQUENCE: SEQ ID NO: 4:
   AGCTATAACT TCGTATAATG TATGCTATAC GAAGTTATCT CGAGA                45
(2) INFORMATION POUR LA SEQ ID NO: 5:
   (i) CARACTERISTIQUES DE LA SEQUENCE:
      (A) LONGUEUR: 45 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: oligonucleotide de synthese (loxP)
      (B) SOUCHE: oTG6522
   (xi) DESCRIPTION DE LA SEQUENCE: SEQ ID NO: 5:
   AGCTTCTCGA GATAACTTCG TATAGCATAC ATTATACGAA GTTAT 45